# FASCICULE DE BREVET EUROPEEN

(11) **EP 1 879 539 B1**
(45) Date de publication et mention de la délivrance du brevet: **25.07.2018**
(21) Numéro de dépôt: 06755464.2
(22) Date de dépôt: 28.04.2006
(51) Int. Cl.: A61K 6/00

(54) **COMPOSITION A BASE D'EXTRAITS DE GRAINES D'ARAUCARIA POUR LA PROTECTION DE LA PEAU**
HAUTSCHUTZZUSAMMENSETZUNG AUF DER BASIS VON ARAUKARIENSAMENEXTRAKTEN
SKIN PROTECTIVE COMPOSITION BASED ON ARAUCARIA GRAIN EXTRACTS

(30) Priorité: 29.04.2005 FR 0504370; 04.05.2005 FR 0504571
(43) Date de publication de la demande: 23.01.2008
(73) Titulaire: SOCIETE DE RECHERCHE COSMETIQUE SARL, 2314 Luxembourg (LU)
(72) Inventeur: LECLERE, Jacques, F-45500 Saint-gondon (FR)
(74) Mandataire: Goulard, Sophie
(86) Numéro de dépôt international: PCT/FR2006/000964
(87) Numéro de publication internationale: WO 2006/117464

(56) Documents cités:
- WO-A-02/069992
- WO-A-2004/019961
- WO-A-2006/066355
- FR-A- 2 810 242
- FR-A- 2 856 298
- FR-A1- 2 849 374
- JP-A- 2002 161 043
- US-A1- 2002 132 021
- YAMAGUCHI ET AL: "Biflavonoids from Brazilian pine Araucaria angustifolia as potentials protective agents against DNA damage and lipoperoxidation" PHYTOCHEMISTRY, PERGAMON PRESS, GB, vol. 66, no. 18, septembre 2005 (2005-09), pages 2238-2247, XP005076935 ISSN: 0031-9422
- MACHADO E L ET AL: "Endogenous protein phosphorylation and casein kinase activity during seed development in Araucaria angustifolia", PHYTOCHEMISTRY, PERGAMON PRESS, GB, vol. 61, no. 7, 1 December 2002 (2002-12-01), pages 835-842, XP004394271, ISSN: 0031-9422, DOI: 10.1016/S0031-9422(02)00429-6
- WAGHORN J J ET AL: "The role of two isoenzymes of alpha-amylase of Araucaria Araucana on the digestion of starch granules during germination", JOURNAL OF EXPERIMENTAL BOTANY, OXFORD UNIVERSITY PRESS, GB, vol. 54, no. 384, 1 January 2003 (2003-01-01), pages 901-911, XP002363257, ISSN: 0022-0957, DOI: 10.1093/JXB/ERG093
- CORDENUNSI B R ET AL: "Chemical composition and glycemic index of Brazilian pine (Araucaria angustifolia) seeds", MEDICINAL & AROMATIC PLANTS ABSTRACTS, SCIENTIFIC PUBLISHERS, NEW DELHI - INDIA, vol. 26, no. 5, 1 October 2004 (2004-10-01), XP018002532,

## Description

La présente invention concerne une nouvelle composition cosmétique et/ou dermatologique destinée à assurer la protection de l'épiderme, et plus particulièrement une composition à base d'extrait de graines d'Araucaria présentant un effet de protection de la peau contre les agressions climatiques, notamment en température élevée, ou contre les signes du vieillissement cutané.

L'araucaria est un arbre de la famille des araucariacées possédant un tronc droit de taille très variable et souvent élevée, jusqu'à 25 à 30 m voire davantage pour certaines espèces, dont les branches portent des feuilles persistantes en forme d'écailles acérées. Cette famille de plante est très ancienne puisqu'on en trouve des traces à la limite du Trias c'est-à-dire à une période dont l'ancienneté est de plusieurs millions d'années.

Les principales espèces d'araucarias sont *Araucaria araucana,* parfois dénommée *Araucaria imbricata,* que l'on trouve principalement au Chili, *Araucaria angustifolia (ou Araucaria brasiliensis) originaire du Brésil et de l'Argentine, Araucaria heterophylla, Araucaria bidwilli, Araucaria cunninghami, Araucaria cooki, Araucaria luxurians,* et *Araucaria excelsa.* Les araucarias sont généralement exploités dans la construction et la décoration et on utilise alors de préférence des espèces implantées dans des zones tempérées et subtropicales facilitant la culture et l'exploitation. Diverses substances utiles ont été extraites de certaines espèces d'araucarias, et par exemple le brevet JP 7025719 décrit un antimicrobien contenant une résine extraite du tronc d'*Araucaria angustifolia,* tandis que le brevet EP 95133 décrit des dérivés polyprénylés utiles dans la synthèse de substances à but thérapeutique, obtenus par extraction de feuilles d'araucariacées, en particulier *Araucaria brasiliana.*

On sait aussi que les graines d'araucaria sont parfois consommées dans l'alimentation, plus particulièrement dans certaines régions d'Amérique du Sud, de la même manière que les pignons de pin. La composition chimique de graines d'*Araucaria brasiliensis* a d'ailleurs été étudiée afin de comprendre leurs propriétés nutritionnelles (B.R. Cordenunsi et al., J. Agric., Food Chem., 2004, 52, 3412-3416).

Des études ont par ailleurs porté sur les mécanismes enzymatiques se produisant dans les graines d'*Araucaria araucana* (J.J. Waghorn et al., Journal of Expérimental Botany, Vol. 54, n°384, 2003, 901-911) et dans les graines d'*Araucaria angustifolia* (E.L. Machado et al., Phytochemistry, 61, 2002, 835-842) .

La peau constitue un véritable organe comprenant plusieurs couches intégrées, allant de la couche superficielle, l'épiderme, jusqu'aux couches plus profondes, le derme et l'hypoderme, et chacune possède des propriétés spécifiques permettant à l'ensemble de réagir et de s'adapter aux conditions de son environnement.

L'épiderme, principalement composé de kératinocytes (90% des cellules épidermiques), de mélanocytes (2 à 3% des cellules épidermiques) et des cellules de Langerhans, a une épaisseur variable selon les différentes parties du corps. Etant donné qu'il constitue la couche externe de la peau, l'épiderme joue un rôle fondamental pour assurer la protection et le maintien d'une bonne trophicité. C'est pourquoi de nombreuses compositions ont été mises au point afin de le protéger et d'améliorer ses fonctions, et notamment de renforcer son élasticité et sa fermeté.

Le derme, plus épais, solide, riche en nerfs, en vaisseaux sanguins et en glandes sudoripares, se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur. En fait, la capacité de la peau à remplacer le collagène endommagé diminue avec le temps, et des espaces et des irrégularités apparaissent dans le réseau du collagène. A l'échelle de la peau, le vieillissement provoque notamment une diminution des synthèses protéiques (collagène, élastine), une diminution de la synthèse des protéoglycanes entre autres, ainsi qu'une élévation des métalloprotéinases de type MMP3.

Bien que de nombreuses compositions dermatologiques et cosmétiques aient été proposées, il existe toujours un besoin de pouvoir disposer de nouvelles compositions topiques alternatives permettant de lutter efficacement contre les effets du vieillissement cutané, et notamment des compositions topiques à base d'extraits végétaux appropriés provenant plus particulièrement de plantes connues pour leurs propriétés favorables.

Les études effectuées par la demanderesse ont montré qu'il était possible de protéger l'épiderme et d'agir efficacement contre les agressions du climat et de l'environnement, notamment contre les effets de l'exposition à la chaleur et aux variations de température, en utilisant des compositions topiques à base d'extraits d'Araucaria.

La présente invention a donc pour objet une nouvelle composition cosmétique et/ou dermatologique à base d'Araucaria, et plus particulièrement à base d'extrait de graines d'Araucaria.

La présente invention a également pour objet une nouvelle composition cosmétique et/ou dermatologique à base d'extraits de graines d'araucaria, comprenant en outre des polyphénols extraits de cacao et des extraits de maca.

Une telle composition présente d'excellentes propriétés utilisables en cosmétique et en dermatologie pour la protection de la peau, contre les signes du vieillissement cutané.

La présente invention a également pour objet l'utilisation d'un extrait de graines d'Araucaria pour la préparation d'une composition topique destinée à lutter contre les agressions du climat et de l'environnement, notamment contre les effets de l'exposition à la chaleur et aux variations de température.

La présente invention a encore pour objet un procédé cosmétique pour combattre les effets des agressions du climat et de l'environnement sur la peau, consistant à appliquer sur les zones de la peau concernées une composition topique contenant une quantité efficace de l'extrait de graines d'Araucaria selon la présente invention.

L'invention a également pour objet l'utilisation d'un extrait de graines d'Araucaria pour la préparation d'un médicament dermatologique pour la protection de l'épiderme contre les agressions de l'environnement et du climat.

La présente invention a aussi pour objet l'utilisation d'une association d'extraits de graines d'araucaria, de polyphénols extraits de cacao et d'extraits de maca pour la préparation d'une composition topique destinée à lutter contre les signes du vieillissement cutané.

La présente invention a encore pour objet un procédé cosmétique pour combattre les effets du vieillissement cutané, consistant à appliquer sur les zones de la peau concernées une composition topique contenant une quantité efficace de l'association ci-dessus.

L'invention a enfin pour objet l'utilisation d'une association d'extraits de graines d'araucaria, de polyphénols extraits de cacao et d'extraits de maca, pour la préparation d'un médicament dermatologique pour la protection de l'épiderme contre les signes du vieillissement cutané.

Les compositions suivant la présente invention se distinguent en ce qu'elles comprennent un extrait de graines d'Araucaria en une quantité efficace pour procurer une protection de l'épiderme, ainsi que des supports et excipients acceptables en dermatologie et en cosmétologie, lesdits excipients étant choisis parmi les agents de pénétration, les épaississants, les émollients, les tensioactifs, les émulsifiants, les conservateurs, les colorants et les parfums.

Ainsi, les compositions topiques de l'invention à base d'extraits de graines d'Araucaria peuvent être utilisées avantageusement en dermatologie et en cosmétologie pour le traitement ou la prévention de dérèglements liés à un stress cutané résultant d'une variation de température, et plus particulièrement pour prévenir ou réduire les effets de l'exposition à la chaleur et aux variations de température.

Les graines d'araucaria sont choisies avantageusement parmi les graines de *Araucaria araucana (Araucaria imbricata), Araucaria angustifolia, Araucaria heterophylla, Araucaria bidwilli, Araucaria cunninghami, Araucaria cooki, Araucaria luxurians,* et *Araucaria excelsa.* On utilise de préférence *Araucaria araucana* et *Araucaria cunninghami.*

Les essais effectués par la demanderesse ont montré que, parmi les diverses parties de la plante, il est préférable d'utiliser les graines, qui sont facilement disponibles et dont la conservation ne soulève généralement pas de difficulté technique.

Les extraits de graines d'Araucaria utilisés dans les compositions suivant la présente invention sont de préférence sous forme d'extrait hydroglycolique de la graine pulvérisée, par exemple dans le butylène glycol-1,3, le butylène glycol-1,4, le pentyl glycol, le capryl glycol, le bipropylène glycol, l'éthoxydiglycol, et la glycérine.

Suivant une forme avantageuse de réalisation, l'extrait utilisable dans l'invention est obtenu par macération à partir de graines d'Araucaria réduites en poudre.

Suivant une forme préférentielle de réalisation, les graines d'araucaria sont broyées et mises à macérer dans l'eau, puis après expression et filtration l'extrait est stabilisé avec le glycol. Suivant une variante, les graines réduites en poudre sont mises à macérer et le macérat est épuisé par un mélange eau / glycol (50/50), puis le produit est décanté, exprimé, clarifié et filtré.

L'extrait (*Araucaria araucana*) dans un mélange butylène glycol - eau (50/50) se présente sous la forme d'un liquide de couleur entre jaune et ambre, d'odeur caractéristique, soluble dans l'eau, se caractérisant par :
- matières sèches 0,64%
- densité à 22°C 1,014
- indice de réfraction à 22°C 1,391
- pH 6,17

Les extraits de graines d'Araucaria inclus dans les compositions suivant la présente invention ont montré dans des tests in vitro des effets de protection de l'épiderme vis-à-vis du stress induit par des écarts importants de température.

Les tests ont été effectués en utilisant des échantillons dosés à 0,64% d'extrait, sur des épidermes reconstitués (Skinethic®) maintenus à température constante (20°C et 42°C).

Les résultats, détaillés ci-après, ont mis en évidence l'effet anti-stress sans effet secondaire néfaste, et en particulier :
- une parfaite innocuité vis-à-vis des épidermes ;
- un effet de diminution significative du taux des protéines de stress HSP70 par comparaison avec le témoin positif à 42°C.

La diminution du taux des protéines de stress HSP 70 a été observée dès 20°C et elle est significative à 42°C. Ce résultat a été confirmé par l'analyse immuno-histochimique sur des coupes d'épidermes reconstitués.

Ces résultats ont été obtenus avec un extrait de graines d'araucaria (Araucaria araucana), et confirment que, en application topique, un tel extrait, ou une composition le contenant, présente un effet de protection vis-à-vis du stress induit par agressions climatiques et plus particulièrement des écarts de températures. Les mêmes résultats sont confirmés avec un extrait sec et un lyophilisat.

Les résultats sont explicités dans les exemples ci-après.

Les compositions selon la présente invention peuvent se présenter sous toutes les formes galéniques usuelles adaptées à une application topique, et de préférence sous forme de lotion, crème, lait ou sérum.

Elles peuvent comprendre entre 0,1% et 20% en poids d'extrait de graines d'araucaria, tel que défini ci-dessus, par rapport au poids total de la composition, et de préférence entre 1 et 10% en poids. Le choix de la dose dans la composition peut être fait en fonction de l'utilisation envisagée. Pour un traitement protecteur prolongé, on utilise de préférence des doses plus faibles, sous forme de lait ou de crème dosée à environ 1 à 5%, tandis qu'un traitement ponctuel peut nécessiter des doses plus élevées, par exemple un sérum dosé entre 5 et 10%.

Comme indiqué ci-dessus, les compositions selon l'invention peuvent contenir, outre un extrait de graines d'araucaria, des polyphénols extraits de cacao et des extraits de maca.

On sait que les polyphénols d'origine végétale, extraits par exemple du raisin ou du cacao, ont des propriétés intéressantes utilisables en cosmétique. Par exemple, le brevet EP 1.289.491 enseigne que les polyphénols extraits du cacao (Theobroma cacao) possèdent une activité anti-rides utile pour les soins contre le vieillissement de la peau. Les polyphénols de cacao se répartissent en trois groupes qui appartiennent tous à la famille des flavonoïdes : les catéchines (flavanols, 37% env.) et notamment la (-)épicatéchine, les procyanidines (oligomères flavanoliques, 58%) et les anthocyanes (5%).

La maca (*Lepidium meyenii Walpers* ou *Lepidium peruvianumm Chacon*) est une plante de la famille des brassicaceae, que l'on trouve généralement dans des zones sud-américaines d'altitude élevée, notamment sur les hauts plateaux des Andes ; *Lepidium peruvianum* pousse exclusivement au Pérou. La maca est particulièrement adaptée aux altitudes élevées, voisines de 3.600 à 4.800 m, et aux basses températures, et on a observé qu'elle pousse mieux aux basses températures. Elle a cependant pu être transplantée dans des régions plus tempérées et plusieurs espèces ont été cultivées dans des pays d'Europe de l'Est (voir K. Valentova et al., "Smallanthus Sonchifolius and Lepidium Meyenii" Biomed Papers 147, p. 119-130 (2003)). Toutefois, il semble que des conditions climatiques froides soient plus favorables à son développement. Elle est aujourd'hui couramment cultivée dans des exploitations agricoles artisanales, la semence étant faite à l'automne et la récolte en début d'été.

Cette plante comporte une racine pivot souterraine de taille généralement comprise entre 3 et 5 cm, surmontée d'un hypocotyle plus large et d'une partie aérienne en forme de rosette comportant 10 à 20 feuilles qui restent en contact avec le sol. Un seul plant de maca peut produire environ 14 g de graines. Le tubercule, constitué de la racine et de la base de l'hypocotyle, est consommé dans l'alimentation des régions productrices des Andes en tant que légume bouilli, frit ou plus souvent cuit. Sa valeur nutritive est proche de celle des céréales les plus courantes des compositions alimentaires usuelles.

La maca a longtemps été considérée comme un aliment procurant des effets bénéfiques sur la santé, et notamment un effet dynamisant, fortifiant et revitalisant. Elle a aussi été souvent présentée comme capable d'augmenter les capacités intellectuelles et physiques des individus, de stimuler la mémoire et la concentration, de réduire la fatigue, de renforcer le système immunologique, de réguler le cycle menstruel, de réduire le syndrome prémenstruel, et d'augmenter la spermatogenèse. Ces propriétés attribuées à la maca expliquent qu'elle soit parfois appelée, à tort, le ginseng péruvien.

L'analyse chimique de la maca a été faite notamment par A. Dini et al., "Chemical Composition of Lepidium Meyenii" Food Chem., 49, 347-349 (1994) et par M. Comas et al., "Estudio Bromatologico de la Maca o Paca (Lepidium Meyenii)" Alimentaria, Madrid, pp. 85-90 (oct. 1997).

La maca brute est généralement utilisée sous forme de poudre, présentée commercialement en gélules dont la consommation suggérée est de l'ordre de 4 g par jour, soit environ 65 mg par kg de poids corporel pour un individu adulte.

La poudre de maca est pratiquement insoluble dans l'eau ce qui explique qu'elle ait été très peu utilisée dans des compositions à but cosmétique ou dermatologique. Des compositions pharmaceutiques contenant des polysaccharides et des amino-acides dérivés de *Lepidium meyenii* sont décrites dans la demande de brevet WO 00.51548. Un extrait peptidique hydrosoluble de maca, obtenu à partir de tubercules broyés, susceptible d'être utilisé dans des compositions cosmétiques pour lutter contre le vieillissement de la peau, est décrit dans le brevet FR 2.856.298.

Pour lutter contre le vieillissement de la peau, on a proposé divers traitements à base de compositions telles que crèmes et lotions contenant des alpha-hydroxyacides ou des rétinoïdes, appliquées régulièrement pour réduire progressivement le nombre de rides et ridules. On a aussi proposé des implants de collagène pour dissimuler les lignes d'expression autour des yeux ou de la bouche, la dermabrasion et les peelings chimiques pour éliminer la couche supérieure de la peau endommagée, la chirurgie esthétique, telle que la blépha-roplastie (chirurgie des paupières) ou un lifting pour rajeunir une peau présentant un affaissement, ou encore une restructuration à l'aide d'un laser au dioxyde de carbone pour éliminer les ridules.

Bien que de nombreuses compositions dermatologiques et cosmétiques aient été proposées, il existe toujours un besoin de pouvoir disposer de nouvelles compositions topiques alternatives permettant de lutter efficacement contre les effets du vieillissement cutané, et notamment des compositions topiques à base d'extraits végétaux appropriés provenant plus particulièrement de plantes, capables de procurer une activité hydratante de la peau, de diminuer les rides, de diminuer les taches pigmentaires, d'améliorer la régénération cutanée et d'induire une augmentation de l'épaisseur de l'épiderme, sans entraîner aucun effet toxique.

Les études effectuées par la demanderesse ont montré qu'il était possible de protéger l'épiderme et d'agir efficacement contre les signes liés au vieillissement cutané, en utilisant des compositions topiques associant des polyphénols extraits de cacao, des extraits de maca et des extraits d'araucaria. Les études ont montré en effet que les effets anti-vieillissement cutané des polyphénols de cacao se trouvaient potentialisés par l'addition d'extraits végétaux provenant de maca et d'araucaria.

Ces compositions se distinguent en ce qu'elles comprennent une association de polyphénols extraits de cacao, d'extraits de maca, et d'extraits de graines d'araucaria en une quantité efficace pour procurer une protection de l'épiderme contre les signes du vieillissement, ainsi que des supports et excipients acceptables en dermatologie et en cosmétologie.

Ainsi, ces compositions topiques peuvent être utilisées avantageusement en dermatologie et en cosmétologie pour le traitement ou la prévention des signes du vieillissement cutané tels que ridules, rides, etc.

L'extrait polyphénolique utilisé dans l'association ci-dessus est de préférence un extrait obtenu par broyage de fèves de cacao et séparation hydrophile/lipophile du beurre de cacao d'une part et d'un mélange de protéines et de polyphénols d'autre part. La séparation hydrophile / lipophile permet, par extraction de la graine broyée, de séparer à l'eau les corps gras surnageants de la phase polyphénols / protéines. On utilise de préférence une eau portée à une température comprise entre 80 et 100°C environ.

Au lieu d'utiliser des extraits de polyphénols de cacao, on peut aussi utiliser des cellules de cacao obtenues par des techniques connues de croissance ou multiplication cellulaire, en milieu liquide ou solide, de cellules d'origine végétale cultivées *in vitro* dans un environnement contrôlé, dans des conditions aseptiques en l'absence de micro-organismes. Ainsi, on pourra se référer aux travaux effectués sur des suspensions cellulaires de cacao tels que ceux de Gurney et Evans, Exp. Bot. 43, 769-775, (1992), Wen et Kinsella, J. Food Sc. 57, 1452-1457 (1992) et Leathers et Scragg, Plant Sc. 62, 217-228 (1989). On peut plus particulièrement utiliser des cellules dédifférenciées lyophilisées de cacao dont les teneurs des trois principaux composants sont respectivement de 1,2 mg d'anthocyanes, 136,0 mg d'oligomères procyanidoliques (OPC) et 11,2 mg d'épicatéchine, par gramme de cellules lyophilisées.

Les extraits de maca utilisés dans les compositions suivant la présente invention sont de préférence sous forme d'extraits hydroglycoliques, par exemple dans le butylène glycol, de la racine réduite en poudre.

Suivant une forme avantageuse de réalisation, l'extrait de maca utilisable est obtenu à partir de racines de maca réduites en poudre, que l'on fait macérer dans un mélange de glycol et d'eau, par exemple un mélange 50/50 de glycol et d'eau, puis on procède à une décantation, une clarification et une filtration. La clarification se fait généralement par centrifugation ou par addition d'une substance absorbant les microparticules, comme la cellulose, la polyvinyl pyrolidone ou des terres absorbantes. Le glycol peut être choisi parmi le butylène glycol-1,3, le butylène glycol-1,4, le dipropylène glycol, et on utilise de préférence le butylène glycol.

Typiquement, les extraits de polyphénols de cacao et de maca détaillés ci-après peuvent être utilisés pour la 'préparation de l'association selon la présente invention, en les associant à l'extrait d'araucaria dont la composition est détaillée plus haut.

### Extrait de polyphénols de cacao

L'extrait est obtenu en effectuant une extraction d'un type connu, par exemple par broyage de fèves de cacao, de préférence à partir des fèves de cacaoyer (Theobroma cacao), et séparation hydrophile / lipophile du beurre de cacao d'une part et d'un mélange de protéines et de polyphénols d'autre part. La séparation hydrophile / lipophile permet, par extraction de la graine broyée, de séparer à l'eau les corps gras surnageants de la phase polyphénols / protéines.

Les protéines sont séparées par exemple par ultrafiltration tangentielle (protéines > 10.000 Da) tandis que les polyphénols sont séchés, puis encapsulés si nécessaire. La fraction enrichie en insaponifiables est obtenue à partir du beurre de cacao par distillation moléculaire à 200-300°C sous vide (environ 10⁻⁴ à 10⁻² mm Hg / 0,13 à 13 Pa).

L'extrait polyphénolique ainsi obtenu se présente sous forme d'un liquide de couleur sombre soluble dans l'eau et les alcools. Il est utilisé de préférence sous forme encapsulée assurant une meilleure protection contre l'oxydation.

### Extrait de maca

L'extrait hydroglycolique de maca utilisé dans la présente invention se présente sous la forme d'un liquide de couleur jaune ambrée, d'odeur caractéristique, soluble dans l'eau et dans l'alcool, se caractérisant par :
- extrait butylène glycol / eau (50/50)
- matières sèches) 4,0 %
- teneur en flavonoïdes 0,04%
- densité à 22°C 1,038
- indice de réfraction à 22°C 1,398
- pH 5,77

Les extraits de maca utilisés dans l'association de l'invention contiennent des isoflavones qui ont été dosées par chromatographie HPLC en phase inverse. On a en particulier identifié les isoflavones constituées par la daidzine, la genistine, la daidzéine et la genistéine. Comme indiqué ci-dessus la teneur en isoflavones par rapport à la matière sèche est de 0,04% pour une teneur moyenne en matières sèche de 5,33% et une dose totale d'isoflavones de 23,42 mg/l. Les isoflavones identifiées dans l'extrait sont des substances connues pour exercer un effet biologique proche de celui de la DHEA, qui peut constituer un effet complémentaire avantageux dans le cadre de l'invention.

La composition selon l'invention comprend généralement entre 0,1 et 2% en poids d'extrait polyphénolique de cacao, entre 1 et 10% en poids d'extrait de maca et entre 1 et 10% en poids d'extrait de graines d'araucaria par rapport au poids total de la composition. Suivant une forme préférentielle, ces concentrations sont respectivement de 0,2 à 1% en poids d'extrait polyphénolique de cacao, 2 à 5% en poids d'extrait de maca et 3 à 6% en poids d'extrait de graines d'araucaria par rapport au poids total de la composition. Dans le cas de l'utilisation de cellules de cacao au lieu d'extrait polyphénoliques de cacao, la concentration en cellules de cacao est généralement comprise entre 0,01 et 0,2% et de préférence entre 0,02 et 0,1%.

L'association de polyphénols extraits de cacao, d'extraits de maca, et d'extraits de graines d'araucaria suivant la présente invention, utilisée dans des conditions normales d'emploi pendant 20 à 30 jours consécutifs, a démontré une excellente tolérance cutanée et s'est avérée non comédogène et non acnéigène.

De plus, les essais effectués en utilisant des compositions sous forme de crèmes ou de sérums et comprenant l'association de l'invention ont mis en évidence une augmentation significative du taux d'hydratation de la peau.

Les essais effectués sur des épidermes reconstitués, traités pendant 48 heures, ont aussi montré, comme indiqué plus loin :
- une absence de cytotoxicité, les images histologiques, après coloration HES, d'épidermes traités par l'association de l'invention étant similaires à celles des épidermes témoins.
- une augmentation de la prolifération des cellules de la couche basale, traduisant une stimulation de la régénération épidermique.
- une diminution de la différenciation épidermique.
- une augmentation de l'épaisseur des épidermes.

Les résultats sont explicités dans les exemples ci-après.

Ils démontrent que l'association suivant l'invention procure des effets anti-vieillissement de l'épiderme potentialisés par rapport à ceux procurés par un extrait de polyphénol seul.

Les tests d'acceptabilité des compositions suivant l'invention par un groupe de 100 femmes les ayant appliquées pendant une période de deux mois ont fait apparaître une appréciation très favorable pour les formulations testées, à savoir une crème de jour, une crème de nuit et un sérum, telles que décrites dans les exemples ci-après. Ces résultats concernent non seulement la facilité d'utilisation que l'efficacité de la composition pour la réduction des rides et la diminution des taches pigmentaires.

Ces compositions selon la présente invention peuvent se présenter sous toutes les formes galéniques usuelles adaptées à une application topique, et de préférence sous forme de lotion, crème, lait ou sérum.

Le choix de la concentration en chacun des composants dans la composition peut être fait en fonction de l'utilisation envisagée. Pour un traitement prolongé, on utilise de préférence des doses plus faibles, sous forme de lait ou de crème dosée à environ 3 à 6% (total des trois composants), tandis qu'un traitement ponctuel peut nécessiter des doses plus élevées, par exemple un sérum dosé entre 6 et 10%, où, de préférence, les doses d'extraits de maca et d'araucaria sont plus élevées.

L'extrait de graines d'araucaria selon l'invention, ou l'association de polyphénols extraits de cacao, d'extraits de maca, et d'extraits de graines d'araucaria suivant la présente invention, peuvent être utilisés seuls ou en association avec d'autres principes actifs complémentaires compatibles, c'est-à-dire non susceptibles de réagir les uns sur les autres ou de masquer ou limiter leurs effets respectifs.

Suivant une forme avantageuse de réalisation, l'extrait de graines d'araucaria suivant l'invention, ou l'association, est complété par des principes actifs ou ingrédients auxiliaires choisis pour leurs propriétés complémentaires, afin respectivement, pour l'extrait d'araucaria seul, de renforcer l'effet d'inhibition des contractions musculaires faciales ou de compléter les effets protecteurs de la composition, et, pour l'association, de renforcer l'effet de protection contre les effets du vieillissement cutané.

Comme actif complémentaire, on peut utiliser par exemple un produit exerçant un effet décontractant sur la peau, comme par exemple un extrait de Lotus bleu, de coquelicot ou de guimauve ou des extraits d'Hibiscus esculentus disponibles dans le commerce sous la marque Myoxinol®.

L'effet protecteur des extraits d'araucaria de l'invention peut aussi être avantageusement complété par des polyphénols extraits de cacao ou de raisin, ou par des produits favorisant la microcirculation cutanée tels que des flavonoïdes et des extraits de mélèze comme le Cryocytol®.

L'effet anti-âge de l'association de l'invention peut également être avantageusement complété par des produits favorisant la microcirculation cutanée tels que des flavonoïdes et des extraits de mélèze comme le Cryocytol®. On peut encore avantageusement incorporer à la composition des facteurs de pénétration et de drainage comme le sulfate de dextran, des extraits de roses de Jericho pour protéger de la déshydratation, de l'acide hyaluronique ou son sel de sodium, des peptides stimulant l'ATP ou encore une substance à effet anti-hyaluronidase telle que l'echinacine B.Les compositions conformes à la présente invention peuvent être présentées sous les formes classiquement utilisées pour une application topique, c'est-à-dire sous forme de lotion, gel, émulsion (en particulier crème ou lait), masque, pommade, nanocapsules, liposomes ou encore des patches transdermiques, contenant des excipients et supports usuels compatibles et pharma-ceutiquement acceptables. Elles peuvent aussi se présenter sous forme de lingettes imbibées d'une solution contenant l'extrait de graines d'araucaria ou, le cas échéant, d'une solution contenant les extraits polyphénoliques de cacao, de maca et de graines d'araucaria. Ces formes d'administration par voie topique sont préparées par les techniques usuelles, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenan pas du tout.

Dans le cas des nanocapsules et des liposomes, il peut être avantageux d'utiliser un extrait aqueux de préférence à un extrait hydroglycolique.

Les compositions topiques selon l'invention comprennent des excipients appropriés pour une administration topique externe, en particulier des excipients acceptables sur le plan dermatologique et cosmétologique. Ces excipients appropriés pour la formulation sont bien connus de l'homme du métier et sont choisis parmi les agents de pénétration tels que l'éthoxydiglycol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsifiants ; les conservateurs tels que le phénoxyéthanol, le parahydroxybenzoate de méthyle (méthylparaben), le parahydroxybenzoate d'éthyle (éthylpara-ben), le parahydroxybenzoate de propyle (propylparaben) et le Phenonip® associant du phénoxyéthanol et des parahydroxy-benzoates de méthyle, éthyle, butyle et isobutyle ; les colorants et les parfums.

D'autres ingrédients peuvent être utilisés dans les compositions : les agents hydratants tels que le propylène glycol, la glycérine, le butylène glycol et également les vitamines antioxydantes telles que la vitamine E, par exemple l'acétate de tocophérol ou le tocotriénol, la vitamine C, les polyphénols naturels. On peut également ajouter à la composition des agents conditionneurs de la peau tels que le nylon et le nitrure de bore, ainsi que des agents de protection contre les rayons ultra-violets, et par exemple des filtres solaires UV-A et UV-B hydrophiles ou lipophiles, choisis parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane. On peut aussi utiliser des pigments formant écran anti-ultraviolet, comme par exemple le dioxyde de titane, l'oxyde de zinc, l'oxyde de zirconium ou encore l'oxyde d'aluminium.

Les exemples suivants illustrent l'invention plus en détail sans en limiter la portée. Dans tous les exemples de compositions qui suivent, les parties sont exprimées en poids, sauf indication contraire.

### Exemple 1

Suivant les techniques classiques, on prépare une crème de soins pour peau sensible ayant la composition pondérale suivante.

| | |
|---|---|
| Huile de coprah hydrogénée | 3,00 |
| Alcool béhénylique | 2,00 |
| Squalane | 5,00 |
| Huile de bourrache | 3,00 |
| Monostéarate de glycérol | 3,00 |
| B.H.A. | 0,05 |
| Eau déminéralisée | qsp 100,00 |
| EDTA trisodique | 0,10 |
| Glycérine | 4,00 |
| Butylène glycol-1,3 | 4,00 |
| Extrait glycolique de bardane | 1,00 |
| Extrait glycolique de camomille | 1,00 |
| Glycérrhétinate de sodium | 0,10 |
| Conservateurs | 0,80 |
| Extrait de graines d'*Araucaria araucana* | 5,00 |

L'extrait de graines d'*Araucaria araucana* utilisé dans la composition ci-dessus est un extrait glycolique obtenu par traitement de graines d'Araucaria araucana broyées et réduites en poudre.

La crème ayant la composition indiquée ci-dessus est utilisée en application sur le visage, une à deux fois par jour.

### Exemple 2

Suivant une technique usuelle, on prépare une crème de protection solaire ayant la composition pondérale suivante.

| | |
|---|---|
| Eau déminéralisée | qsp 100,00 |
| EDTA trisodique | 0,10 |
| Glycérine | 4,00 |
| Butylène glycol-1,3 | 4,00 |
| Conservateurs | 0,70 |
| Chlorphénésine | 0,05 |
| Gomme xanthane | 0,15 |
| Benzoate d'alkyle C12-C15 | 10,00 |
| Huile de coprah hydrogénée | 3,50 |
| Palmitate de cétyle | 1,50 |
| Dilinoléate de di-isopropyle dimère | 6,00 |
| Phényltriméthicone | 2,00 |
| Stéarate de sorbitane | 2,50 |
| PEG 20 stéarate de sorbitane | 3,50 |
| Ceteareth 20 | 0,20 |
| Beurre de karité | 3,00 |
| Méthoxycinnamate d'octyle (Parsol MCX®) | 2,50 |
| Salicylate d'octyle | 3,00 |
| Butylméthoxydibenzoyl méthane | 2,50 |
| Extrait de graines d'*Araucaria araucana* | 6,00 |

### Exemple 3

Suivant les techniques classiques, on prépare une lotion tonique ayant la composition pondérale suivante.

| | |
|---|---|
| Eau déminéralisée | qsp 100,00 |
| Conservateurs | 0,50 |
| EDTA trisodique | 0,05 |
| Glycérine | 3,00 |
| Butylène glycol-1,3 | 4,00 |
| Lactate de sodium | 2,00 |
| Urée | 0,20 |
| Eau de roses | 20,00 |
| Eau de tilleul | 20,00 |
| Tween 20 | 0,50 |
| α-bisabolol | 0,05 |
| alcool éthylique à 96% vol. | 5,00 |
| Glycérrhétinate de sodium | 0,10 |
| Extrait de graines d'*Araucaria araucana* | 10,00 |

### Exemple 4

Suivant les techniques classiques, on prépare une crème de soins (crème de nuit) ayant la composition pondérale suivante.

| | |
|---|---|
| Eau déminéralisée | qsp 100,00 |
| Butylène glycol-1,3 | 5,40 |
| Glycérine | 4,60 |
| Dilinoléate de di-isopropyle dimère | 4,00 |
| Octyl dodécanol | 3,00 |
| Huile de coprah hydrogénée | 2,60 |
| Palmitate de glycol | 2,40 |
| Phosphate d'alkyle C20-C22 | 2,50 |
| Alcools C20-C22 | 2,00 |
| Méthacrylate de polyméthyle | 1,50 |
| Phényl triméthicone | 1,70 |
| Dimethicone | 0,90 |
| Polyacrylamide | 0,35 |
| Tocophérol | 0,50 |
| Trométhamine | 0,25 |
| Tristéarine | 0,25 |
| Chlorphénésine | 0,10 |
| Sulfate de dextrane | 0,20 |
| Cellules de cacao | 0,05 |
| Extrait de Lepidium meyenii (racine) | 3,00 |
| Extrait de graines d'*Araucaria araucana* | 4,00 |
| Conservateurs | 0,60 |

La crème ayant la composition indiquée ci-dessus est utilisée en application sur le visage, une fois par jour au coucher pendant une période de 1 à 3 mois.

### Exemple 5

Suivant une technique usuelle, on prépare une crème anti-âge (crème de jour) ayant la composition pondérale suivante.

| | |
|---|---|
| Eau déminéralisée | qsp 100,00 |
| Dilinoléate de di-isopropyle dimère | 6,00 |
| Méthoxy cinnamate d'éthylhexyle | 6,00 |
| Butylène glycol-1,3 | 5,00 |
| Glycérine | 4,00 |
| Neopentanoate d'isodécyle | 4,00 |
| Salicylate d'éthylhexyle | 3,00 |
| Huile de coprah hydrogénée | 3,00 |
| Phosphate d'alkyle C20-C22 | 2,60 |
| Alcools C20-C22 | 2,00 |
| Butylméthoxy dibenzoylméthane | 2,50 |
| Palmitate de glycol | 1,50 |
| Xylitol | 0,60 |
| Méthacrylate de polyméthyle | 0,50 |
| Phényl triméthicone | 1,50 |
| Polyacrylamide | 0,30 |
| Tocophérol | 0,50 |
| Trométhamine | 0,30 |
| Tristéarine | 0,25 |
| Chlorphénésine | 0,10 |
| Hyaluronate de sodium | 0,05 |
| Cellules de cacao | 0,05 |
| Extrait de Lepidium meyenii (racine) | 4,00 |
| Extrait de graines d'*Araucaria araucana* | 4,00 |
| Conservateurs | 0,60 |
| Parfum | 0,50 |

La crème ayant la composition indiquée ci-dessus est utilisée en application sur le visage, une à deux fois par jour pendant une période de 1 à 3 mois.

### Exemple 6

Suivant les techniques classiques, on prépare un sérum ayant la composition pondérale suivante.

| | |
|---|---|
| Eau déminéralisée | qsp 100,00 |
| Butylène glycol-1,3 | 7,50 |
| Succinate de diéthylhexyle | 6,00 |
| Glycérine | 4,70 |
| Octyl dodécanol | 3,00 |
| Xylitol | 0,60 |
| Tocophérol | 0,50 |
| Polyacrylate de sodium | 1,20 |
| Phényl triméthicone | 1,50 |
| Chlorphénésine | 0,10 |
| Gomme xanthane | 0,01 |
| Céramide 3 | 0,03 |
| Céramide 6 | 0,02 |
| EDTA tétrasodique | 0,10 |
| Extrait phénolique de cacao | 0,50 |
| Extrait de Lepidium meyenii (racine) | 5,00 |
| Extrait de graines d'*Araucaria araucana* | 5,00 |
| Conservateurs | 0,70 |
| Parfum | 0,60 |

### Exemple 7

L'étude des effets de l'extrait de graine d'araucaria suivant la présente invention sur la protection de l'épiderme vis-à-vis du stress induit par des écarts de température a été faite sur un modèle in vitro d'épidermes reconstitués, comme indiqué plus haut.

La protection épidermique est évaluée par dosage des protéines de stress HSP 70, qui sont des protéines libérées à la suite d'un stress résultant d'une irradiation aux ultra-violets, et par marquage immunohistochimique.

### Protocole expérimental

L'étude a été réalisée sur des épidermes reconstitués (Skinethic®). Chaque essai a été réalisé en triplicate.

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air-liquide, le milieu de culture étant changé tous les deux jours.

Les épidermes ainsi formés ont été utilisés pour la réalisation de l'étude à partir du 17^{éme} jour de culture.

### Essai préliminaire

L'essai est conduit en triplicate après 6 heures de traitement (contact des extraits hydroglycoliques d'araucaria avec les épidermes). Quatre lots ont été constitués.
- lot 1 : épiderme témoin ne recevant aucun produit
- lot 2 : épiderme traité par l'extrait d'Araucaria araucana
- lot 3 : épiderme maintenu à la température de 20°C
- lot 4 : épiderme maintenu à la température de 42°C

Les épidermes fixés dans une solution de formaldéhyde à 10% sont inclus dans des blocs de paraffine. Les coupes verticales de 4 microns sont colorées à l'hématoxyline / éosine (HES) et photographiés sous un microscope.

L'interprétation histo-pathologique se fait de la manière suivante. Les cultures doivent présenter des couches cellulaires basales, spineuses, granuleuses et cornées intactes, orthokératosiques, et la stratification épidermique doit être régulière et normale. Les cellules de la couche basale doivent être polarisées verticalement. De nombreux grains de kératohyaline doivent être visibles (de couleur violette) dans la couche granuleuse, juste sous la couche cornée.

### Evaluation de l'effet anti-stress

L'essai est conduit en triplicate après 6 heures de contact entre l'échantillon de produit et l'épiderme reconstitué. 6 lots sont préparés :
- lot 1 : épiderme témoin ne recevant aucun produit
- lot 2 : épiderme maintenu à la température de 20°C
- lot 3 : épiderme maintenu à la température de 42°C
- lot 4 : épiderme traité par l'extrait d'Araucaria araucana
- lot 5 : épiderme maintenu à la température de 20°C et traité par l'extrait d'Araucaria araucana
- lot 6 : épiderme maintenu à la température de 42°C et traité par l'extrait d'Araucaria araucana

L'extrait d'Araucaria araucana est utilisé à raison de 2 µl/cm².

Les protéines de stress HSP 70 ont été quantifiées par un dosage immunologique à l'aide d'un anticorps spécifique antihumain (Stressgen®). La révélation a été réalisée au moyen d'un anticorps secondaire, puis la réaction a été arrêtée par addition d'une solution acide. La mesure de l'absorbance a été faite à 450nm.

Par ailleurs, à la fin du temps d'incubation (6 heures) les épidermes ont été fixés dans une solution de formol, puis déshydratés par des bains successifs d'alcool. Après inclusion, les épidermes ont été coupés et traités en immuno-histochimie. Cette réaction a été réalisée par l'anticorps monoclonal anti-HSP 70 (Sigma).

Les résultats sont regroupés dans les tableaux ci-dessous. L'évaluation de l'effet anti-stress des protéines HSP 70 sans induction par la variation de température fournit les résultats suivants :

| | HSP 70 (pg/ml) | % |
|---|---|---|
| Témoin | 1325 ± 183 | - |
| Extrait d'Araucaria | 1281 ± 143 | ns |

L'évaluation de l'effet anti-stress des protéines HSP 70 après induction par la variation de température fournit les résultats suivants :

| | HSP 70 (pg/ml) | % |
|---|---|---|
| Témoin | 1325 ± 183 | - |
| T = 20°C | 1519 ± 127 | +15 (ns) |
| T = 42°C | 1897 ± 165 | +43 |
| T = 20°C + Extrait d'Araucaria | 1420 ± 97 | -7 (ns) |
| T = 42°C + Extrait d'Araucaria | 1508 ± 112 | -21 |

Ces résultats montrent que l'incubation des épidermes à 20°C et à 42°C entraîne une augmentation des protéines de stress HSP 70. Le traitement des épidermes par l'extrait d'araucaria suivant l'invention induit une diminution significative des HSP 70 après incubation à 42°C.

De plus, il a été montré que l'extrait d'Araucaria araucana suivant l'invention, déposé à raison de 2 µg/cm², sur des épidermes reconstitués traités pendant 24 heures, comparativement à des épidermes témoins, n'ont induit aucune toxicité. Les images histologiques, après coloration HES, sont comparables pour les épidermes traités et pour les épidermes témoins.

Ces résultats mettent en évidence l'effet protecteur de l'épiderme exercé par l'extrait de graines d'araucaria de l'invention.

### Exemple 8

L'étude des effets de l'association suivant la présente invention sur la protection de l'épiderme vis-à-vis des signes du vieillissement cutané, par hydratation et régénération épidermique, a été faite sur un modèle in vitro d'épidermes reconstitués (Skinethic®).

### Protocole expérimental

L'étude a été réalisée sur des épidermes reconstitués (Skinethic®). Chaque essai a été réalisé en triplicate.

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air-liquide, le milieu de culture étant changé tous les deux jours.

Les épidermes ainsi formés ont été utilisés pour la réalisation de l'étude à partir du 14^{ème} jour de culture.

### Essai préliminaire

L'essai, destiné à déterminer le temps de contact produit / épiderme, a été conduit en duplicate après 48 heures de traitement (contact de l'association d'extrait polyphénolique de cacao, d'extraits de maca et d'extraits d'araucaria, avec les épidermes).

Deux lots ont été constitués.
- lot 1 : 3 épidermes témoins ne recevant aucun produit
- lot 2 : épidermes traités par l'association de l'invention.

Les épidermes fixés dans une solution de formaldéhyde à 10% sont inclus dans des blocs de paraffine. Les coupes verticales de 4 microns sont colorées à l'hématoxyline / éosine (HES) et photographiés sous un microscope.

L'interprétation histo-pathologique se fait comme indiqué dans l'exemple 7.

### Evaluation de la viabilité cellulaire

On a constaté que la composition de l'invention, déposée à raison de 2 µl/cm² sur les épidermes reconstitués traités pendant 48 heures, n'induit aucune toxicité par rapport au lot témoin. Après coloration HES, les images histologiques des épidermes traits sont similaires à celles des épidermes témoins.

Les épidermes traités présentent des couches cellulaires basales, spineuses, granuleuses et cornées intactes. La stratification épidermique est régulière et normale. Le cellules de la couche basale sont polarisées verticalement. Enfin, de nombreux grains de kératohyaline sont visibles dans la couche granuleuse juste sous la couche cornée.

Une étude clinique sur 20 femmes volontaires a confirmé la très bonne tolérance cutanée de la composition. L'étude a été effectuée en administrant la composition sous forme de sérum sur les jambes des patientes pendant 28 jours consécutifs.

### Evaluation de l'index mitotique

L'index mitotique a été évalué après traitement immuno-histochimique des épidermes reconstitués.

Le nombre de cellules marquées après comptage des sites nucléaires colorés en brun de tous les échantillons traduisant un index mitotique est indiqué dans le tableau ci-après.

| | Nombre de cellules marquées | % |
|---|---|---|
| Témoin | 17,2 ± 0,8 | - |
| Composition de l'invention | 22, 0 ± 1,0 | +27 |

### Evaluation du marquage de la filaggrine

Le marquage de la filaggrine après traitement immuno-histochimique des épidermes reconstitués a été évalué. L'observation comparative des épidermes reconstitués (épidermes témoins et épidermes traités par la composition de l'invention) a montré que, dans les conditions physiologiques, les épidermes témoins présentent un marquage intense et uniforme de la filaggrine au niveau de la couche granuleuse, tandis que les épidermes traités présentent un marquage moins intense mais uniforme de la filaggrine.

### Evaluation de la densité des épidermes reconstitués

L'épaisseur moyenne des épidermes reconstitués (épidermes témoins et épidermes traités par la composition de l'invention) est reportée dans le tableau ci-après.

| | Epaisseur moyenne (µm) | % |
|---|---|---|
| Témoin | 132 ± 6 | - |
| Invention (sérum) | 147 ± 5 | +11 |
| Invention (crème de jour) | 150 ± 5 | +13 |
| Invention (crème de nuit) | 156 ± 5 | +18 |

On constate donc que la composition de l'invention induit une stimulation de l'épiderme reconstitué se traduisant par une augmentation significative de son épaisseur, due principalement à la régulation des processus de prolifération cellulaire et de différentiation, sans aucune toxicité vis-à-vis de l'épiderme.

### Effet hydratant

Les trois compositions de l'invention décrites dans les exemples 4, 5 et 6 (crème de nuit, crème de jour et sérum) ont été testées sur des volontaires afin de vérifier la variation du taux d'hydratation cutanée après une application standardisée unique de la composition, en comparaison avec un zone non traitée.

Les essais ont montré une augmentation significative du taux d'hydratation 1 heure après application, qui est encore sensible après 8 heures comme le montre le tableau ci-après.

| | variation % | |
|---|---|---|
| | t + 1 heure | t + 8 heures |
| sérum | +48 ± 7 | +28 ± 5 |
| crème de jour | +40 ± 9 | +21 ± 5 |
| crème de nuit | +40 ± 7 | +23 ± 2 |

L'étude a été faite sur 10 volontaires, âgées entre 23 et 64 ans, en une application standardisée unique (2 µl/cm²), la composition étant appliquée sur les jambes.

## Revendications

1. Composition cosmétique et/ou dermatologique pour application topique, destinée à assurer la protection de la peau, **caractérisée en ce qu'**elle comprend une quantité efficace d'un extrait de graines d'araucaria, et des excipients acceptables sur le plan dermatologique et cosmétologique, lesdits excipients étant choisis parmi les agents de pénétration, les épaississants, les émollients, les tensioactifs, les émulsifiants, les conservateurs, les colorants et les parfums.

2. Composition selon la revendication 1, **caractérisée en ce que** l'extrait est obtenu à partir de graines de *Araucaria araucana (Araucaria imbricata), Araucaria angustifolia, Araucaria heterophylla, Araucaria bidwilli, Araucaria cunninghami, Araucaria cooki, Araucaria luxurians,* et *Araucaria excelsa.*

3. Composition selon la revendication 2, **caractérisée en ce que** l'extrait est sous forme hydroglycolique.

4. Composition selon la revendication 3, **caractérisée en ce que** l'extrait est obtenu par macération à partir de graines d'araucaria (*Araucaria araucana ou Araucaria cunninghami*) réduites en poudre.

5. Composition selon l'une quelconque des revendications précédentes, **caractérisée en ce que** l'extrait de graines d'araucaria est un liquide **se caractérisant par** :
- matières sèches 0,64%
- densité à 22°C 1,014
- indice de réfraction à 22°C 1,391
- pH 6,17

6. Composition selon l'une quelconque des revendications précédentes, **caractérisée en ce qu'**elle comprend entre 0,1 et 20% en poids d'extrait de graines d'araucaria.

7. Composition selon la revendication 6, **caractérisée en ce qu'**elle comprend entre 1 et 10% en poids d'extrait de graines d'araucaria.

8. Composition selon l'une quelconque des revendications 1 à 7, **caractérisée en ce qu'**elle comprend en outre des polyphénols extraits de cacao et des extraits de maca.

9. Composition selon la revendication 8, **caractérisée en ce que** l'extrait de maca est obtenu par extraction hydroglycolique à partir de racines de maca réduites en poudre.

10. Composition selon la revendication 8, **caractérisée en ce que** l'extrait polyphénolique est obtenu par broyage de fèves de cacao et séparation hydrophile/lipophile à l'eau.

11. Composition selon l'une quelconque des revendications 8 à 10, **caractérisée en ce qu'**elle comprend entre 0,1 et 2% en poids d'extrait polyphénolique de cacao, entre 1 et 10% en poids d'extrait de maca et entre 1 et 10% en poids d'extrait de graines d'araucaria par rapport au poids total de la composition.

12. Composition selon la revendication 11, **caractérisée en ce qu'**elle comprend entre 0,2 et 1% en poids d'extrait polyphénolique de cacao, 2 à 5% en poids d'extrait de maca et 3 à 6% en poids d'extrait de graines d'araucaria par rapport au poids total de la composition.

13. Composition selon la revendication 8, **caractérisée en ce qu'**elle comprend des cellules de cacao en une concentration comprise entre 0,01 et 0,2% en poids par rapport au poids total de la composition.

14. Utilisation d'un extrait de graines d'araucaria pour la préparation d'une composition topique destinée à lutter contre les agressions du climat et de l'environnement.

15. Procédé cosmétique pour combattre les effets des agressions du climat et de l'environnement sur la peau, consistant à appliquer sur les zones de la peau concernées une composition contenant une quantité efficace de la composition topique selon l'une quelconque des revendications 1 à 7.

16. Utilisation d'un extrait de graines d'araucaria pour la préparation d'un médicament dermatologique pour la protection de l'épiderme contre les agressions de l'environnement et du climat.

17. Utilisation d'une association d'extraits de graines d'araucaria, de polyphénols extraits de cacao et d'extraits de maca, pour la préparation d'une composition topique destinée à lutter contre les signes du vieillissement cutané.

18. Procédé cosmétique pour combattre les effets du vieillissement cutané, consistant à appliquer sur les zones de la peau concernées une composition contenant une quantité efficace de la composition topique selon l'une quelconque des revendications 8 à 13.

19. Utilisation d'une association d'extraits de graines d'araucaria, de polyphénols extraits de cacao et d'extraits de maca pour la préparation d'un médicament dermatologique pour la protection de l'épiderme contre les signes du vieillissement cutané.

## Patentansprüche

1. Kosmetische und/oder dermatologische Zusammensetzung zur topischen Anwendung, ausgelegt, um den Schutz der Haut sicherzustellen, **dadurch gekennzeichnet, dass** sie eine wirksame Menge eines Araucariensamenextrakts und annehmbare Hilfsstoffe auf dermatologischer und kosmetologischer Ebene umfasst, wobei die Hilfsstoffe ausgewählt sind aus den Eindringmitteln, den Verdickungsmitteln, den Weichmachern, den Tensiden, den Emulgatoren, den Konservierungsstoffen, den Farbstoffen und den Parfüms.

2. Zusammensetzung nach Anspruch 1, **dadurch gekennzeichnet, dass** der Extrakt erhalten wird auf der Basis von Samen von *Araucaria araucana (Araucaria imbricata), Araucaria angustifolia, Araucaria heterophylla, Araucaria bidwilli, Araucaria cunninghami, Araucaria cooki, Araucaria luxurians* und *Araucaria excelsa.*

3. Zusammensetzung nach Anspruch 2, **dadurch gekennzeichnet, dass** der Extrakt eine hydroglycolische Form aufweist.

4. Zusammensetzung nach Anspruch 3, **dadurch gekennzeichnet, dass** der Extrakt durch Mazeration auf der Basis von Araucariensamen (*Araucaria araucana* oder *Araucaria cunninghami*), reduziert zu Pulver, erhalten wird.

5. Zusammensetzung nach einem beliebigen der vorhergehenden Ansprüche, **dadurch gekennzeichnet, dass** der Araucariensamenextrakt eine Flüssigkeit ist, **gekennzeichnet durch**:
- Trockenmassen 0,64 %
- Dichte bei 22 °C 1,014
- Brechungsindex bei 22 ºC 1,391
- pH 6,17

6. Zusammensetzung nach einem beliebigen der vorhergehenden Ansprüche, **dadurch gekennzeichnet, dass** sie zwischen 0,1 und 20 Gew% Araucariensamenextrakt umfasst.

7. Zusammensetzung nach Anspruch 6, **dadurch gekennzeichnet, dass** sie zwischen 1 und 10 Gew% Araucariensamenextrakt umfasst.

8. Zusammensetzung nach einem beliebigen der Ansprüche 1 bis 7, **dadurch gekennzeichnet, dass** sie außer den Polyphenolen Kakaoextrakte und Macaextrakte umfasst.

9. Zusammensetzung nach Anspruch 8, **dadurch gekennzeichnet, dass** der Macaextrakt durch hydroglykolische Extraktion auf der Basis von Macawurzeln, reduziert zu Pulver, erhalten wird.

10. Zusammensetzung nach Anspruch 8, **dadurch gekennzeichnet, dass** der polyphenolishe Extrakt durch Mahlen von Kakaobohnen und hydrophile/liophile Trennung mit Wasser erhalten wird.

11. Zusammensetzung nach einem beliebigen der Ansprüche 8 bis 10, **dadurch gekennzeichnet, dass** sie zwischen 0,1 und 2 Gew% polyhenolischen Kakaoextrakt, zwischen 1 und 10 Gew% Macaextrakt und zwischen 1 und 10 Gew% Araucariensamenextrakt mit Bezug auf das Gesamtgewicht der Zusammensetzung umfasst.

12. Zusammensetzung nach Anspruch 11, **dadurch gekennzeichnet, dass** sie zwischen 0,2 und 1 Gew% polyhenolischen Kakaoextrakt, 2 bis 5 Gew% Macaextrakt und 3 bis 6 Gew% Araucariensamenextrakt mit Bezug auf das Gesamtgewicht der Zusammensetzung umfasst.

13. Zusammensetzung nach Anspruch 8, **dadurch gekennzeichnet, dass** sie Kakaozellen in einer Konzentration umfasst, die im Bereich zwischen 0,01 und 0,2 % mit Bezug auf das Gesamtgewicht der Zusammensetzung liegt.

14. Verwendung eines Araucariensamenextrakts zur Herstellung einer topischen Zusammensetzung, die ausgelegt ist, um gegen die Aggressionen des Klimas und der Umwelt zu kämpfen.

15. Kosmetisches Verfahren zur Bekämpfung der Wirkungen der Aggressionen des Klimas und der Umwelt auf die Haut, das darin besteht, auf die betroffenen Bereiche der Haut eine Zusammensetzung aufzubringen, die eine wirksame Menge der topischen Zusammensetzung nach einem beliebigen der Ansprüche 1 bis 7 enthält.

16. Verwendung eines Araucariensamenextrakts zur Herstellung eines dermatologischen Medikaments zum Schutz der Epidermis gegen die Agressionen der Umwelt und des Klimas.

17. Verwendung einer Verbindung von Araucariensamenextrakten von Polyphenolen, extrahiert von Kakao, und Macaextrakten für die Herstellung einer topischen Zusammensetzung, die ausgelegt ist, um gegen die Zeichen der Alterung der Haut zu kämpfen.

18. Kosmetisches Verfahren, um die Wirkungen der Alterung der Haut zu bekämpfen, das darin besteht, auf die betroffenen Bereiche der Haut eine Zusammensetzung aufzubringen, die eine wirksame Menge der topischen Zusammensetzung nach einem beliebigen der Ansprüche 8 bis 13 enthält.

19. Verwendung einer Verbindung von Araucariensamenextrakten, von Polyphenolen, extrahiert von Kakao, und Macaextrakten zur Herstellung eines dermatologischen Medikaments zum Schutz der Epidermis gegen die Zeichen der Alterung der Haut.

## Claims

1. Cosmetic and/or dermatological composition for topical application, intended to ensure the protection of the skin, **characterized in that** it comprises an effective amount of an extract of araucaria seeds, as well as excipients that are dermatologically and cosmetologically acceptable, wherein the excipients are chosen from penetrating agents, thickeners, emollients, surfactants, emulsifiers, preservatives, dyes and perfumes.

2. Composition according to claim 1, **characterized in that** the extract is obtained from seeds of Araucaria araucana (Araucaria imbricata), Araucaria angustifolia, Araucaria heterophylla, Araucaria bidwilli, Araucaria cunninghami, Araucaria cooki, Araucaria luxurians, and Araucaria excelsa.

3. Composition according to claim 2, **characterized in that** the extract is in a glycolic form.

4. Composition according to claim 3, **characterized in that** the extract is obtained by maceration of araucaria seeds (Araucaria araucana or Araucaria cunninghami) ground to powder.

5. Composition according to any one of the preceding claims, **characterized in that** the araucaria seed extract is a liquid **characterized by**
- dry matter 0.64%
- density of 1.014 at 22°C
- refractive index of 1.391 at 22°C
- pH of 6.17

6. Composition according to any one of the preceding claims, **characterized in that** it comprises between 0.1 and 20 % by weight of araucaria seed extract.

7. Composition according to claim 6, **characterized in that** it comprises between 1 and 10 % by weight of araucaria seed extract.

8. Composition according to any one of the claims 1 to 7, **characterized in that**
it further comprises polyphenols extracted from cocoa and extracts of maca.

9. Composition according to claim 8, **characterized in that** the maca extract is obtained by hydroglycolic extraction from powdered maca roots.

10. Composition according to claim 8, **characterized in that** the polyphenol extract is obtained by grinding cocoa beans and hydrophilic/lipophilic separation with water.

11. Composition according to any one of the claims 8 to 10, **characterized in that** it comprises between 0.1 and 2 % by weight of polyphenol extract of cocoa, between 1 and 10% by weight of extract of maca, and between 1 and 10% by weight of araucaria seed extract, relative to the total weight of the composition.

12. Composition according to claim 11, **characterized in that** it comprises between 0.2 and 1 % by weight of cocoa polyphenol extract, 2 to 5 % by weight of maca extract and 3 to 6 % by weight of araucaria seed extract, relative to the total weight of the composition.

13. Composition according to claim 8, **characterized in that** it comprises cocoa cells in a concentration of between 0.01 and 0.2 % by weight relative to the total weight of the composition.

14. Use of araucaria seed extract for the preparation of a topical composition for combating climatic and environmental aggressions.

15. Cosmetic method for combating the effects of climatic and environmental aggressions on the skin, comprising applying to the areas of the skin concerned, a composition containing an effective amount of the topical composition according to any one of the claims 1 to 7.

16. Use of an extract of araucaria seeds for the preparation of a dermatological medicament for the protection of the epidermis against environmental and climatic aggressions.

17. Use of a combination of Araucaria seed extracts, polyphenols extracted from cocoa, and extracts of maca, for the preparation of a topical composition for combating the signs of skin aging.

18. Cosmetic method for combating the effects of skin aging, comprising applying to the areas of the skin concerned, a composition containing an effective amount of the topical composition according to any one of the claims 8 to 13.

19. Use of a combination of Araucaria seed extract, cocoa extract polyphenols and maca extract for the preparation of a dermatological medicament for the protection of the epidermis against signs of skin aging.
